# FASCICULE DE BREVET EUROPEEN

(11) **EP 2 403 529 B1**
(45) Date de publication et mention de la délivrance du brevet: **31.12.2014**
(21) Numéro de dépôt: 10715299.3
(22) Date de dépôt: 05.03.2010
(51) Int. Cl.: A61K 39/395, C07K 16/34, A61P 37/00

(54) **ANTICORPS MONOCLONAL ANTI-RHESUS D**
MONOKLONALER ANTI-RHESUS-D-ANTIKÖRPER
ANTI-RHESUS D MONOCLONAL ANTIBODY

(30) Priorité: 06.03.2009 FR 0951412
(43) Date de publication de la demande: 11.01.2012
(73) Titulaire: Laboratoire Français du Fractionnement et des Biotechnologies, 91940 Les Ulis (FR)
(72) Inventeur: GAUCHER, Christine, 59320 Sequedin (FR); JORIEUX, Sylvie, 59650 Villeneuve d'Ascq (FR); DE ROMEUF, Christophe, 59130 Lambersart (FR)
(74) Mandataire: Chajmowicz, Marion
(86) Numéro de dépôt international: PCT/FR2010/050376
(87) Numéro de publication internationale: WO 2010/100383

(56) Documents cités:
- WO-A-01/77181
- WO-A-2005/040216
- WO-A2-2008/121615
- SIBERIL S ET AL: "Selection of a human anti-RhD monoclonal antibody for therapeutic use: Impact of IgG glycosylation on activating and inhibitory FcgammaR functions" CLINICAL IMMUNOLOGY, ACADEMIC PRESS, US, vol. 118, no. 2-3, 1 février 2006 (2006-02-01), pages 170-179, XP024911799 ISSN: 1521-6616 [extrait le 2006-02-01] cité dans la demande
- BELIARD ROLAND ET AL: "A human anti-D monoclonal antibody selected for enhanced Fc gamma RIII engagement clears RhD+ autologous red cells in human volunteers as efficiently as polyclonal anti-D antibodies" BRITISH JOURNAL OF HAEMATOLOGY, vol. 141, no. 1, avril 2008 (2008-04), pages 109-119, XP002545262 ISSN: 0007-1048
- KIPRIYANOV S M ET AL: "Two amino acid mutations in an anti-human CD3 single chain Fv antibody fragment that affect the yield on bacterial secretion but not the affinity" PROTEIN ENGINEERING, OXFORD UNIVERSITY PRESS, SURREY, GB, vol. 10, no. 4, 1 janvier 1997 (1997-01-01), pages 445-453, XP002237690 ISSN: 0269-2139
- ZHANG WEI ET AL: "Free sulfhydryl in recombinant monoclonal antibodies" BIOTECHNOLOGY PROGRESS, AMERICAN INSTITUTE OF CHEMICAL ENGINEERS, US, vol. 18, no. 3, 1 mai 2002 (2002-05-01), pages 509-513, XP002500112 ISSN: 8756-7938
- OSTERMEIER CHRISTIAN ET AL: "Crystals of an Antibody F-v Fragment Against an Integral Membrane Protein Diffracting to 1.28 A Resolution" PROTEINS STRUCTURE FUNCTION AND GENETICS, vol. 21, no. 1, 1995, pages 74-77, XP009122619 ISSN: 0887-3585
- BANKS DOUGLAS D ET AL: "Removal of cysteinylation from an unpaired sulfhydryl in the variable region of a recombinant monoclonal IgG1 antibody improves homogeneity, stability, and biological activity." JOURNAL OF PHARMACEUTICAL SCIENCES FEB 2008, vol. 97, no. 2, février 2008 (2008-02), pages 764-779, XP002545264 ISSN: 0022-3549
- SCHMIEDL ET AL: "Effects of unpaired cysteines on yield, solubility and activity of different recombinant antibody constructs expressed in E. coli" JOURNAL OF IMMUNOLOGICAL METHODS, ELSEVIER SCIENCE PUBLISHERS B.V.,AMSTERDAM, NL, vol. 242, no. 1-2, 28 août 2000 (2000-08-28), pages 101-114, XP004210714 ISSN: 0022-1759
- ZHANG WEI ET AL: "Complete disulfide bond assignment of a recombinant immunoglobulin G4 monoclonal antibody." ANALYTICAL BIOCHEMISTRY, vol. 311, no. 1, 1 décembre 2002 (2002-12-01), pages 1-9, XP002545265 ISSN: 0003-2697
- DAUGHERTY A L ET AL: "Formulation and delivery issues for monoclonal antibody therapeutics" ADVANCED DRUG DELIVERY REVIEWS, ELSEVIER BV, AMSTERDAM, NL LNKD- DOI:10.1016/J.ADDR.2006.03.011, vol. 58, no. 5-6, 7 août 2006 (2006-08-07) , pages 686-706, XP024892149 ISSN: 0169-409X [extrait le 2006-08-07]

<110> LFB Biotechnologies
<120> Anticorps monoclonal anti-Rhésus D
<130> B849
<160> 11
<170> PatentIn version 3.3
<210> 1
   <211> 1425
   <212> DNA
   <213> Homo sapiens
<220>
   <221> CDS
   <222> (58)..(1425)
<400> 1
   atggagtttg ggctgagctg ggttttcctc gttgctcttt taagaggtgt ccagtgt         57
   cag gtg cag ctg gtg gag tct ggg gga ggc gtg gtc cag cct ggg agg       105
   Gln Val Gln Leu Val Glu Ser Gly Gly Gly Val Val Gln Pro Gly Arg
   1               5                   10                  15
   tcc ctg aga ctc tcc tgt aca gcc tct gga ttc acc ttc aaa aac tat       153
   Ser Leu Arg Leu Ser Cys Thr Ala Ser Gly Phe Thr Phe Lys Asn Tyr
               20                  25                  30
   gct atg cat tgg gtc cgc cag gct cca gcc aag ggg ctg gag tgg gtg       201
   Ala Met His Trp Val Arg Gln Ala Pro Ala Lys Gly Leu Glu Trp Val
           35                  40                  45
   gca act ata tca tat gat gga agg aat ata caa tat gca gac tcc gtg       249
   Ala Thr Ile Ser Tyr Asp Gly Arg Asn Ile Gln Tyr Ala Asp Ser Val
       50                  55                  60
   aag ggc cga ttc acc ttc tcc aga gac aat tct cag gac acc ctg tat       297
   Lys Gly Arg Phe Thr Phe Ser Arg Asp Asn Ser Gln Asp Thr Leu Tyr
   65                  70                  75                  80
   ctg caa ctg aac agc ctc aga ccg gag gac acg gct gtg tat tac tgt       345
   Leu Gln Leu Asn Ser Leu Arg Pro Glu Asp Thr Ala Val Tyr Tyr Cys
                   85                  90                  95
   gcg aga ccc gta aga agc cga tgg ctg caa tta ggt ctt gaa gat gct       393
   Ala Arg Pro Val Arg Ser Arg Trp Leu Gln Leu Gly Leu Glu Asp Ala
               100                 105                 110
   ttt cat atc tgg ggc cag ggg aca atg gtc acc gtc tct tca gcc tcc       441
   Phe His Ile Trp Gly Gln Gly Thr Met Val Thr Val Ser Ser Ala Ser
           115                 120                 125
   acc aag ggc cca tcg gtc ttc ccc ctg gca ccc tcc tcc aag agc acc       489
   Thr Lys Gly Pro Ser Val Phe Pro Leu Ala Pro Ser Ser Lys Ser Thr
       130                 135                 140
   tct ggg ggc aca gcg gcc ctg ggc tgc ctg gtc aag gac tac ttc ccc      537
   Ser Gly Gly Thr Ala Ala Leu Gly Cys Leu Val Lys Asp Tyr Phe Pro
   145                 150                 155                 160
   gaa ccg gtg acg gtg tcg tgg aac tca ggc gcc ctg acc agc ggc gtg      585
   Glu Pro Val Thr Val Ser Trp Asn Ser Gly Ala Leu Thr Ser Gly Val
                   165                 170                 175
   cac acc ttc ccg gct gtc cta cag tcc tca gga ctc tac tcc ctc agc      633
   His Thr Phe Pro Ala Val Leu Gln Ser Ser Gly Leu Tyr Ser Leu Ser
               180                 185                 190
   agc gtg gtg acc gtg ccc tcc agc agc ttg ggc acc cag acc tac atc      681
   Ser Val Val Thr Val Pro Ser Ser Ser Leu Gly Thr Gln Thr Tyr Ile
           195                 200                 205
   tgc aac gtg aat cac aag ccc agc aac acc aag gtg gac aag aaa gtt      729
   Cys Asn Val Asn His Lys Pro Ser Asn Thr Lys Val Asp Lys Lys Val
       210                 215                 220
   gag ccc aaa tct tgt gac aaa act cac aca tgc cca ccg tgc cca gca      777
   Glu Pro Lys Ser Cys Asp Lys Thr His Thr Cys Pro Pro Cys Pro Ala
   225                 230                 235                 240
   cct gaa ctc ctg ggg gga ccg tca gtc ttc ctc ttc ccc cca aaa ccc      825
   Pro Glu Leu Leu Gly Gly Pro Ser Val Phe Leu Phe Pro Pro Lys Pro
                   245                 250                 255
   aag gac acc ctc atg atc tcc cgg acc cct gag gtc aca tgc gtg gtg      873
   Lys Asp Thr Leu Met Ile Ser Arg Thr Pro Glu Val Thr Cys Val Val
               260                 265                 270
   gtg gac gtg agc cac gaa gac cct gag gtc aag ttc aac tgg tac gtg      921
   Val Asp Val Ser His Glu Asp Pro Glu Val Lys Phe Asn Trp Tyr Val
           275                 280                 285
   gac ggc gtg gag gtg cat aat gcc aag aca aag ccg cgg gag gag cag      969
   Asp Gly Val Glu Val His Asn Ala Lys Thr Lys Pro Arg Glu Glu Gln
       290                 295                 300
   tac aac agc acg tac cgt gtg gtc agc gtc ctc acc gtc ctg cac cag     1017
   Tyr Asn Ser Thr Tyr Arg Val Val Ser Val Leu Thr Val Leu His Gln
   305                 310                 315                 320
   gac tgg ctg aat ggc aag gag tac aag tgc aag gtc tcc aac aaa gcc     1065
   Asp Trp Leu Asn Gly Lys Glu Tyr Lys Cys Lys Val Ser Asn Lys Ala
                   325                 330                 335
   ctc cca gcc ccc atc gag aaa acc atc tcc aaa gcc aaa ggg cag ccc     1113
   Leu Pro Ala Pro Ile Glu Lys Thr Ile Ser Lys Ala Lys Gly Gln Pro
               340                 345                 350
   cga gaa cca cag gtg tac acc ctg ccc cca tcc cgg gat gag ctg acc     1161
   Arg Glu Pro Gln Val Tyr Thr Leu Pro Pro Ser Arg Asp Glu Leu Thr
           355                 360                 365
   aag aac cag gtc agc ctg acc tgc ctg gtc aaa ggc ttc tat ccc agc     1209
   Lys Asn Gln Val Ser Leu Thr Cys Leu Val Lys Gly Phe Tyr Pro Ser
       370                 375                 380
   gac atc gcc gtg gag tgg gag agc aat ggg cag ccg gag aac aac tac      1257
   Asp Ile Ala Val Glu Trp Glu Ser Asn Gly Gln Pro Glu Asn Asn Tyr
   385                 390                 395                 400
   aag acc acg cct ccc gtg ctg gac tcc gac ggc tcc ttc ttc ctc tac      1305
   Lys Thr Thr Pro Pro Val Leu Asp Ser Asp Gly Ser Phe Phe Leu Tyr
                   405                 410                 415
   agc aag ctc acc gtg gac aag agc agg tgg cag cag ggg aac gtc ttc      1353
   Ser Lys Leu Thr Val Asp Lys Ser Arg Trp Gln Gln Gly Asn Val Phe
               420                 425                 430
   tca tgc tcc gtg atg cat gag gct ctg cac aac cac tac acg cag aag      1401
   Ser Cys Ser Val Met His Glu Ala Leu His Asn His Tyr Thr Gln Lys
           435                 440                 445
   agc ctc tcc ctg tct ccg ggt aaa                                      1425
   Ser Leu Ser Leu Ser Pro Gly Lys
       450                 455
<210> 2
   <211> 456
   <212> PRT
   <213> Homo sapiens
<400> 2
   Gln Val Gln Leu Val Glu Ser Gly Gly Gly Val Val Gln Pro Gly Arg
   1 5 10 15
   Ser Leu Arg Leu Ser Cys Thr Ala Ser Gly Phe Thr Phe Lys Asn Tyr
   20 25 30
   Ala Met His Trp Val Arg Gln Ala Pro Ala Lys Gly Leu Glu Trp Val
   35 40 45
   Ala Thr Ile Ser Tyr Asp Gly Arg Asn Ile Gln Tyr Ala Asp Ser Val
   50 55 60
   Lys Gly Arg Phe Thr Phe Ser Arg Asp Asn Ser Gln Asp Thr Leu Tyr
   65 70 75 80
   Leu Gln Leu Asn Ser Leu Arg Pro Glu Asp Thr Ala Val Tyr Tyr Cys
   85 90 95
   Ala Arg Pro Val Arg Ser Arg Trp Leu Gln Leu Gly Leu Glu Asp Ala
   100 105 110
   Phe His Ile Trp Gly Gln Gly Thr Met Val Thr Val Ser Ser Ala Ser
   115 120 125
   Thr Lys Gly Pro Ser Val Phe Pro Leu Ala Pro Ser Ser Lys Ser Thr
       130                 135                 140
   Ser Gly Gly Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Ser Leu Gly Thr Gln Thr Tyr Ile
           195                 200                 205
   Cys Asn Val Asn His Lys Pro Ser Asn Thr Lys Val Asp Lys Lys Val
       210                 215                 220
   Glu Pro Lys Ser Cys Asp Lys Thr His Thr Cys Pro Pro Cys Pro Ala
   225                 230                 235                 240
   Pro Glu Leu Leu Gly Gly Pro Ser Val Phe Leu Phe Pro Pro Lys Pro
                   245                 250                 255
   Lys Asp Thr Leu Met Ile Ser Arg Thr Pro Glu Val Thr Cys Val Val
               260                 265                 270
   Val Asp Val Ser His Glu Asp Pro Glu Val Lys Phe Asn Trp Tyr Val
           275                 280                 285
   Asp Gly Val Glu Val His Asn Ala Lys Thr Lys Pro Arg Glu Glu Gln
       290                 295                 300
   Tyr Asn Ser Thr Tyr Arg Val Val Ser Val Leu Thr Val Leu His Gln
   305                 310                 315                 320
   Asp Trp Leu Asn Gly Lys Glu Tyr Lys Cys Lys Val Ser Asn Lys Ala
                   325                 330                 335
   Leu Pro Ala Pro Ile Glu Lys Thr Ile Ser Lys Ala Lys Gly Gln Pro
               340                 345                 350
   Arg Glu Pro Gln Val Tyr Thr Leu Pro Pro Ser Arg Asp Glu Leu Thr
           355                 360                 365
   Lys Asn Gln Val Ser Leu Thr Cys Leu Val Lys Gly Phe Tyr Pro Ser
   370 375 380
   Asp Ile Ala Val Glu Trp Glu Ser Asn Gly Gln Pro Glu Asn Asn Tyr
   385 390 395 400
   Lys Thr Thr Pro Pro Val Leu Asp Ser Asp Gly Ser Phe Phe Leu Tyr
   405 410 415
   Ser Lys Leu Thr Val Asp Lys Ser Arg Trp Gln Gln Gly Asn Val Phe
   420 425 430
   Ser Cys Ser Val Met His Glu Ala Leu His Asn His Tyr Thr Gln Lys
   435 440 445
   Ser Leu Ser Leu Ser Pro Gly Lys
   450 455
<210> 3
   <211> 708
   <212> DNA
   <213> Homo sapiens
<220>
   <221> CDS
   <222> (67) .. (708)
<400> 3
<210> 4
   <211> 214
   <212> PRT
   <213> Homo sapiens
<400> 4
<210> 5
   <211> 16
   <212> PRT
   <213> Homo sapiens
<400> 5
   Leu Ser Cys Thr Ala Ser Gly Phe Thr Phe Lys Cys Thr Phe Ser Arg
   1 5 10 15
<210> 7
   <211> 5
   <212> PRT
   <213> Homo sapiens
<400> 7
   Cys Thr Phe Ser Arg
   1 5
<210> 8
   <211> 31
   <212> PRT
   <213> Homo sapiens
<400> 8
   Asp Asn Ser Gln Asp Thr Leu Tyr Leu Gln Leu Asn Ser Leu Arg Pro
   1 5 10 15
   Glu Asp Thr Ala Val Tyr Tyr Cys Ala Arg Cys Thr Phe Ser Arg
   20 25 30
<210> 9
   <211> 26
   <212> PRT
   <213> Homo sapiens
<400> 9
   Asp Asn Ser Gln Asp Thr Leu Tyr Leu Gln Leu Asn Ser Leu Arg Pro
   1 5 10 15
   Glu Asp Thr Ala Val Tyr Tyr Cys Ala Arg
   20 25
<210> 10
   <211> 64
   <212> DNA
   <213> Artificial
<220>
   <223> amorce (primer)
<400> 10
   ctatatcata tgatggaagg aatatacaat atgcagactc cgtgaagggc cgattcacct 60
   tctc 64
<210> 11
   <211> 23
   <212> DNA
   <213> Artificial
<220>
   <223> amorce (primer)
<400> 11
   ggaagtagtc cttgaccagg cag 23

## Description

L'invention concerne un anticorps dirigé contre le Rhésus D (ou anticorps anti-RhD).

### Arrière-plan technologique

On désigne communément sous le terme « Rhésus positif » ou « Rh-positif » les sujets dont les hématies sont agglutinées par des alloanticorps dirigés contre l'antigène D (qui est l'un des antigènes du système RH), et sous le terme « Rhésus négatif», ou « Rh-négatif» les sujets dont les hématies ne sont pas agglutinées par ces alloanticorps.

La maladie hémolytique du nouveau né est due dans la majorité des cas, à la présence, chez une mère Rh-négatif, d'alloanticorps anti-RhD (l'allo- immunisation contre d'autres antigènes du système RH est beaucoup plus rare), qui provoquent chez un foetus Rhésus positif une anémie hémolytique qui va nécessiter, soit des transfusions *in utero,* soit une exsanguinotransfusion à la naissance dans les cas graves.

L'allo-immunisation de la mère intervient généralement lors d'un accouchement précédent; des hématies foetales passent dans la circulation maternelle, et induisent une immunisation si l'enfant est Rh-positif.

La prévention de la maladie hémolytique du nouveau-né consiste en l'injection d'anticorps anti-Rh D, aux femmes Rhésus négatif, immédiatement après un accouchement ou une interruption de grossesse.

Actuellement, les anticorps anti-Rhésus utilisés dans ce but sont des immunoglobulines polyclonales provenant de donneurs volontaires Rhésus négatif, immunisés à plusieurs reprises contre des hématies Rh-positif.

Ceci pose des problèmes, d'une part du fait de la nécessité de disposer de volontaires en nombre suffisant pour répondre aux besoins, et d'autre part du fait des risques de contamination par des virus ou autres pathogènes qui pourraient être présents dans les préparations d'immunoglobulines obtenues à partir du sang des volontaires.

Bien que plusieurs anticorps monoclonaux anti-RhD aient été générés pour remplacer les anticorps polyclonaux, à ce jour aucun n'est encore disponible en clinique (Sibéril et al, Clincial Immunology, 2006, 118 :170-179).

Le clone T125 produit par des cellules YB2/0 de myélome de rat (clone désigné T125 YB2/0), rapporté par Sibéril et al, *supra* (et la demande WO2001/77181), était un candidat prometteur, mais risquant d'être relativement instable en raison de réarrangements intramoléculaires.

### Résumé de l'invention

Les inventeurs ont maintenant mis au point un nouvel anticorps monoclonal anti-RhD, qui présente une stabilité améliorée.

Cet anticorps, qui est une immunoglobuline IgG1, contient une chaîne lourde codée par la séquence nucléotidique SEQ ID N°1. Plus particulièrement, l'anticorps est une immunoglobuline IgG1 tétramérique composée de deux chaînes lourdes et deux chaînes légères, la chaîne lourde comprenant la séquence d'acides aminés SEQ ID N°2, et la chaîne légère comprenant la séquence d'acides aminés SEQ ID N°4.

Cet anticorps est désigné anticorps R593 dans la présente description.

L'anticorps R593 a été obtenu par mutation de l'anticorps désigné R297, issu du clone T125 A2 produit par des lymphocytes B transformés par l'EBV. Comme l'anticorps R297, l'anticorps R593 de l'invention est une IgG1 tetramerique composée de 2 chaînes lourdes et 2 chaînes légères qui possèdent 32 résidus cystéines engagés dans 16 ponts disulfures intra-chaîne lourde (4 par chaîne), intra-chaîne légère (2 par chaîne) et inter-chaîne (4 par chaîne). L'anticorps R593 de l'invention se distingue de l'anticorps R297 par un résidu phénylalanine à la place d'un résidu cystéine en position 68 de la chaîne lourde. Sa spécificité antigénique est aussi bonne, et sa stabilité est améliorée, car aucun réarrangement intramoléculaire indésirable n'est plus possible.

Un autre objet de l'invention est une composition pharmaceutique comprenant ledit anticorps, en association avec des excipients pharmaceutiquement acceptables.
De préférence la composition comprend un tampon citrate.
Avantageusement, la composition comprend également un excipient polyol tel que le mannitol.
De préférence encore, elle comprend un tensioactif non ionique.
Une composition particulièrement préférée comprend l'anticorps associé à un tampon citrate 30mM, pH 6,5, du polysorbate 80 ou un poloxamer, du mannitol et du NaCl. Un exemple de composition comprend un tampon citrate 30mM, pH6,5, 400ppm de polysorbate 80, 17g/L de mannitol et 3,25g/L de NaCl. Un autre exemple de composition comprend un tampon citrate 30mM, pH6,5, 400ppm de polysorbate 80 ou 301 ppm de poloxamer 188, 17g/L de mannitol et 3,25g/L de NaCl.

### Description détaillée de l'invention :

### Production de l'anticorps

L'anticorps monoclonal de l'invention peut être produit par toute technique connue de l'homme du métier, par exemple par recombinaison dans une cellule hôte, transformée avec un ou des vecteur(s) qui permettent l'expression et/ou la sécrétion des séquences nucléotidiques codant pour la chaîne lourde ou la chaîne légère de l'anticorps. Le vecteur comporte généralement un promoteur, des signaux d'initiation et de terminaison de la traduction, ainsi que des régions appropriées de régulation de la transcription. Il est maintenu de façon stable dans la cellule hôte et peut éventuellement posséder des signaux particuliers qui spécifient la sécrétion de la protéine traduite. Ces différents éléments sont choisis et optimisés par l'homme du métier en fonction de l'hôte cellulaire utilisé.

Est donc décrit aussi un acide nucléique codant pour la chaîne lourde d'un anticorps, ladite chaîne lourde comprenant la séquence d'acides aminés SEQ ID N°2.

Il est fourni également un vecteur d'expression, par exemple un vecteur viral ou plasmidique, comprenant un acide nucléique tel que défini ici. Le vecteur peut être à réplication autonome au sein de l'hôte choisi, ou il peut s'agir de vecteurs intégratifs de l'hôte choisi. Est également utile un vecteur d'expression comprenant un acide nucléique codant pour la chaîne légère de l'anticorps. Un autre objet de l'invention est un vecteur d'expression comprenant un acide nucléique codant pour la chaîne lourde et pour la chaîne légère de l'anticorps, tels que définis ici.
De tels vecteurs sont préparés par des méthodes couramment utilisées par l'homme du métier, et les clones résultant peuvent être introduits dans un hôte approprié par des méthodes standard, telles que la lipofection, l'électroporation, l'utilisation d'agents polycationiques, le choc thermique, ou des méthodes chimiques.

Est également décrite une cellule hôte transfectée avec ledit ou lesdits vecteurs. L'hôte cellulaire peut être choisi parmi des systèmes procaryotes ou eucaryotes, par exemple les cellules bactériennes mais également les cellules de levure ou les cellules animales, en particulier les cellules de mammifères. On peut également utiliser des cellules d'insectes ou des cellules de plantes.

Sous un autre aspect, l'invention a pour objet un procédé de production d'un anticorps de l'invention, ledit procédé comprenant les étapes suivantes : a) la culture dans un milieu et conditions de culture appropriés d'une cellule hôte exprimant une chaîne lourde et une chaîne légère telles que définies ici ; et b) la récupération desdits anticorps ainsi produits à partir du milieu de culture ou desdites cellules cultivées.

Un exemple particulier de procédé de production est une production dans une cellule d'insecte, comme décrit par exemple dans la demande internationale WO96/07740. Pour cela, une cassette d'expression comprenant une séquence codant pour la région variable de la chaîne légère de l'anticorps monoclonal, ou une séquence codant pour la région variable de la chaine lourde de l'anticorps monoclonal, laquelle séquence est placée sous contrôle transcriptionnel d'un promoteur approprié, par exemple un promoteur de baculovirus.

A titre d'exemple de promoteurs de baculovirus, on citera les promoteurs de la polyedrine et de P10 des baculovirus AcMNPV ou SIMNPV, ou des dérivés de promoteurs de baculovirus, constitués par des promoteurs synthétiques ou recombinants, obtenus a partir d'un promoteur de baculovirus, et fonctionnels en cellules d'insectes.

La présente Invention fournit également des vecteurs recombinants, portant au moins une cassette d'expression telle que définie ci-dessus; dans ce cadre, la présente invention englobe en particulier des baculovirus recombinants permettant l'expression de l'anticorps R593, ainsi que des plasmides de transfert permettant la construction desdits baculovirus recombinants.
Pour permettre l'expression simultanée de la chaine lourde (chaine H) et de la chaine légère (chaine L) et leur réassociation pour former la molécule d'anticorps recombinant, on peut utiliser deux cassettes sur un même vecteur d'expression. On peut ainsi par exemple préparer un baculovirus double recombinant dans lequel la séquence codante de chacune des chaines H et L est sous le contrôle d'un promoteur fort. Pour cela, on peut suivre les étapes suivantes :
- on prépare séparément deux plasmides de transfert, un pour la chaine H, et un pour la chaine L ;
- on cotransfecte ensuite les cellules d'insecte avec l'ADN des vecteurs de transfert ainsi réalisés et l'ADN du baculovirus. Cette cotransfection est effectuée en deux étapes: Le plasmide de transfert contenant la cassette d'expression pour le gène de la chaine légère flanquée des régions entourant le gène de la polyedrine dans le baculovirus sauvage, est utilisé, avec l'ADN de baculovirus sauvage AcMNPV, pour cotransfecter des cellules d'insecte en culture. Par recombinaison homologue entre l'ADN viral et le plasmide, les séquences codantes de la chaine légère de l'immunoglobuline recombinante sont transférées dans le génome viral.
- Apres réplication de l'ADN viral dans les cellules transfectées, on procède a la sélection des baculovirus recombinants ayant intégré la séquence de la chaine légère de l'immunoglobuline recombinante.
- dans une étape suivante, les cellules sont cotransfectées avec l'ADN du baculovirus recombinant obtenu ci-dessus, et avec celui du plasmide de transfert contenant la cassette d'expression portant le gène codant pour la chaine lourde de l'anticorps recombinant flanquée des régions entourant le gène P10 du baculovirus. Par recombinaison homologue, comme précédemment, le gène de la chaine lourde est transféré dans l'ADN viral.
- on sélectionne alors les virus double- recombinants qui sont capables de produire simultanément une chaine lourde et une chaine légère d'immunoglobuline.

Un autre exemple de procédé de production est l'utilisation d'un vecteur d'expression de type viral ou plasmidique, pour l'expression de l'anticorps monoclonal dans une cellule de mammifère.
Les cellules de mammifère préférées pour l'expression de l'anticorps monoclonal sont la lignée de rat YB2/0, la lignée de hamster CHO, en particulier les lignées CHO dhfr- et CHO Lec13, PER.C6TM (Crucell), 293, K562, NS0, SP2/0, BHK ou COS.

Un autre mode de production est l'expression de l'anticorps recombinant dans des organismes transgéniques, par exemple dans les plantes (Ayala M, Gavilondo J, Rodríguez M, Fuentes A, Enríquez G, Pérez L, Cremata J, Pujol M. Production of plantibodies in Nicotiana plants. Methods Mol Biol. 2009;483:103-34.) ou bien dans le lait d'animaux transgéniques tels que le lapin, la chèvre ou le porc (Pollock, D.P., J.P. Kutzko, E. Birck-Wilson, J.L. Williams, Y. Echelard and H.M. Meade. (1999). Transgenic milk as a method for the production of recombinant antibodies. Journal of Immunological Methods. 231: 147-157).

### Applications thérapeutiques

L'anticorps monoclonal anti-RhD de l'invention est utile à titre de médicament, en particulier pour la prévention la prévention de l'allo-immunisation Rhésus d'individus Rh négatif. Le mode d'action des immunoglobulines anti-D *in vivo* est une fixation spécifique des anticorps sur l'antigène D des globules rouges Rh (D) positif, suivie d'une élimination de ces globules rouges de la circulation essentiellement au niveau de la rate. Cette clairance est associée à un mécanisme dynamique de suppression de la réponse immune primaire chez les individus et prévient donc l'immunisation.
Ainsi l'anticorps de l'invention est particulièrement utile pour la prévention d'une maladie hémolytique chez le nouveau-né, par administration à une femme RhD négatif.
On peut en effet utiliser un anticorps de l'invention de manière prophylactique pour la prévention de l'alloimmunisation de femme Rhésus négatif, immédiatement après la naissance d'un enfant Rhésus positif, et pour prévenir, lors des grossesses ultérieures, la maladie hémolytique du nouveau-né (MHNN) ; lors d'avortements, de grossesses extra-utérines en situation d'incompatibilité Rhésus D ou encore lors d'hémorragies transplacentaires résultant d'amniocentèse, de biopsies chorioniques, ou de manipulations obstétriques traumatisantes en situation d'incompatibilité Rhésus D.

En outre, on peut utiliser un anticorps de l'invention dans le cas de transfusions Rh incompatibles avec du sang ou des dérivés sanguins labiles.

Un anticorps de l'invention est également utile pour la prévention ou le traitement d'un Purpura Thrombocytopénique Idiopathique (PTI).

### Formulations

Un autre objet de l'invention concerne donc une composition pharmaceutique comprenant ledit anticorps à titre de principe actif, en association avec un ou des excipient(s) pharmaceutiquement acceptable(s).

Dans la présente description, on entend désigner par excipient pharmaceutiquement acceptable, un composé ou une combinaison de composés entrant dans une composition pharmaceutique ne provoquant pas de réactions secondaires et qui permet par exemple la facilitation de l'administration du ou des composés actifs, l'augmentation de sa durée de vie et/ou de son efficacité dans l'organisme, l'augmentation de sa solubilité en solution ou encore l'amélioration de sa conservation.

Ces excipients pharmaceutiquement acceptables sont bien connus et seront adaptés par l'homme de l'art en fonction de la nature et du mode d'administration du ou des composés actifs choisis.

De préférence la formulation est conservée sous forme liquide, ou sous forme lyophilisée.

Des substances tampon peuvent être utilisées, par exemple sous forme de carbonate, phosphate, citrate, acetate, borate, trimethamine [(2-amino-2-hydroxymethyl-1,-3-propanediol),TRIS], glycine et lysine (PDA Journal of Pharmaceutical Science and Technology, Vol. 51(4), 1997: Excipients and their use in injectable products (SANDEEP NEMA, R.J. WASHKUHN, R.J. BRENDEL, pp166-171).

Des compositions de l'anticorps dans du tampon citrate (par exemple à environ 30mM) se sont particulièrement montrées stables. Des formulations présentant un pH d'environ 5,5 à moins de 7, de préférence d'environ 6 à environ 6,5, sont préférées.
Les inventeurs ont montré que l'ajout de mannitol et de NaCl augmentait la solubilité de l'anticorps. La quantité de mannitol et NaCl est choisie généralement de manière à obtenir une tonicité de l'ordre de 300mOsm/kg.
L'ajout d'un tensioactif de type polymère non-ionique, comme le polysorbate 80 (Tween ® 80) ou un poloxamer de type poloxamer 188 (Pluronic F68 ® or Lutrol F 68®) est aussi avantageux, par exemple en une quantité d'environ 200 à environ 600 ppm, de préférence d'environ 300 à environ 500ppm, de préférence d'environ 300ppm à environ 400ppm.

L'invention fournit plus particulièrement une composition pharmaceutique comprenant l'anticorps de l'invention, en présence d'un tampon citrate 30mM, pH 6,5, de mannitol, de NaCl, et de polysorbate 80 ou d'un poloxmer, tel que du poloxamer 188.
Une composition pharmaceutique préférée comprend l'anticorps de l'invention, en présence d'un tampon citrate 30mM, pH 6,5, et de polysorbate 80 400ppm ou de poloxamer 188 301 ppm, avec une concentration de mannitol et de NaCl suffisante pour obtenir une tonicité de 300mOsm/Kg.

De manière préférée la composition pharmaceutique comprend d'environ 0,2 à environ 5g/L de l'anticorps, de préférence environ 0,3g/L de l'anticorps.

De préférence, l'anticorps est administré par voie systémique, en particulier par voie intraveineuse, par voie intramusculaire, intradermique, intrapéritonéale ou sous-cutanée, ou par voie orale. De manière plus préférée, la composition comprenant les anticorps selon l'invention, est administrée à plusieurs reprises, de manière étalée dans le temps.

Leurs modes d'administration, posologies et formes galéniques optimaux peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés.

Les exemples et figures suivants illustrent l'invention sans en limiter la portée.

### Légende des figures

La Figure 1 montre la carte de restriction du vecteur T125-H26.
La Figure 2 montre la carte de restriction du vecteur T125-DHFR.
La Figure 3 montre la carte de restriction du vecteur H416-24.
La Figure 4 montre la carte de restriction du vecteur T125-Phe68.
La Figure 5 montre la carte de restriction du vecteur H416-30.
La Figure 6 montre la carte de restriction du vecteur K416-23.
La Figure 7 montre la carte de restriction du vecteur HK463-18.
La Figure 8 est un schéma montrant la construction du vecteur T125-Phe68.

### Exemples

### Exemple 1 : Identification d'une instabilité de l'anticorps R297

L'anticorps R297 est une IgG1 tetramerique composée de 2 chaînes lourdes et 2 chaînes légères qui possèdent 32 résidus cystéines engagés dans 16 ponts disulfures intra-chaîne lourde (4 par chaîne), intra-chaîne légère (2 par chaîne) et inter-chaîne (4). L'anticorps R297 possède également au niveau N-terminal de sa chaîne lourde une cystéine non appariée en position Cys 68. La présence de ce SH libre très réactif au voisinage du pont disulfure intra-chaîne Cys22-Cys96 peut entraîner une compétition et des réarrangements moléculaires avec formation de nouveaux ponts disulfures.
Les inventeurs ont alors identifié les ponts disulfures de R297 et les éventuels réarrangements moléculaires.
Cette étude a été réalisée par cartographie peptidique en conditions non réductrices, de façon à préserver leur intégrité, et identification des peptides générés par spectrométrie de masse MALDI-TOF (Matrix-Assisted Laser Desorption / Ionisation Time of Flight Mass Spectrometry).
Les résultats obtenus ont permis d'identifier les ponts disulfures suivants : Cys23-Cys88, Cys134-Cys194 pour la chaîne légère et Cys22-Cys96, Cys153-Cys209 Cys270-Cys330 et Cys376-Cys434 pour la chaîne lourde.
Les spectres de masse MALDI révèlent la présence d'un peptide de masse 1771.79 Da correspondant au dipeptide [20LSCTASGFTFK30]-[68CTFSR72] (SEQ ID N°5) contenant le pont disulfure Cys22-Cys68 (masse théorique 1771.81 Da). L'analyse MS-MS de l'ion parent à 1771.797 Da permet de confirmer les séquences LSCTASGFTFK (SEQ ID N°6) et CTFSR (SEQ ID N°7) de ce dipeptide. Enfin, par réduction in situ, ce pic diminue au profit de deux ions à 613.28 et 1161.56 Da correspondant aux masses théoriques des peptides [68CTFSR72] (SEQ ID N°7) et [20LSCTASGFTFK30] (SEQ ID N°6) respectivement (masses th : 613.28 et 1161.57 Da). Le peptide contenant la Cys96 non appariée a également été identifié. L'ensemble de ces résultats montre la présence d'un pont disulfure Cys22-Cys68.
De la même façon les spectres MALDI révèlent la présence d'un peptide de masse 3658.54Da correspondant au dipeptide [73DNSQDTLYLQLNSLRPEDTAVYYCAR99]-[68CTFSR72] (SEQ ID N°8) contenant le pont disulfure Cys22-Cys96 (masse théorique 3658.58Da). L'analyse MS-MS de l'ion parent permet de confirmer la séquence du dipeptide. Ce pic diminue après réduction sur cible au profit de deux ions de masses correspondant aux peptides [68CTFSR72] (SEQ ID N°7) et [73DNSQDTLYLQLNSLRPEDTAVYYCAR99] (SEQ ID N°9). Le peptide contenant la Cys22 libre a par ailleurs été identifié. L'ensemble de ces résultats révèle la présence d'un pont disulfure Cys68-Cys96.
L'analyse structurale par MALDI-MS a permis d'identifier tous les ponts disulfures intra-chaîne de R297. On note que la Cys68 non appariée interagit avec le pont disulfure voisin Cys22-Cys96 pour former les ponts Cys22-Cys68 et Cys68-Cys96. Ces réarrangements intramoléculaires peuvent induire des changements de la structure tridimensionnelle de la région N-terminale du Fab et avoir un impact sur l'affinité pour l'antigène et l'immunogénicité de la protéine. La présence de ces différentes formes impliquerait donc une quantification avec un contrôle systématique de la reproductibilité et de la stabilité de celles-ci dans le produit final au cours du développement.

### Exemple 2 : Production d'un anticorps muté

### Matériels et méthodes

Des techniques classiques de biologie moléculaire ont été mises en oeuvre. La mutagenèse a été réalisée par PCR, la région porteuse de la mutation a ensuite été amplifiée par PCR et clonée dans un vecteur intermédiaire. Le vecteur final a été construit par clonage du vecteur chaîne lourde dans le vecteur chaîne légère. Les plasmides recombinants ainsi obtenus ont été ensuite introduits dans des bactéries (transformation des bactéries), puis criblés pour obtenir des séquences conformes à la séquence attendue avant amplification (culture des bactéries) du clone sélectionné afin d'obtenir des quantités de vecteur suffisantes pour l'étape de transfection. Les vecteurs produits au cours de la culture bactérienne ont été ensuite purifiés puis linéarisés en prévision de la transfection dans la lignée YB2/0.
Les amorces utilisées étaient les suivantes :
Amorce A2VH11 5'-CTATATCATATGATGGAAGGAATATACAATATGCAGACTCCGTGAAGGGCC GATCACCTTCTC-3' (SEQ ID N° 10)
   nucléotides soulignés : site de restriction Ndel
   nucléotide encadré : base mutée, mutation du codon TGC (codant pour l'acide aminé Cystéine) à codon TTC (codant pour l'acide aminé phénylalanine)
   Cette amorce 5' localisée dans la région VH de T125 A2 introduit la mutation G à T.
Amorce GSP2ANP
   5'- GGAAGTAGTCCTTGACCAGGCAG -3' (SEQ ID N°11)
   Cette amorce 3' (antisens) est localisée dans la partie 5' de la région constante G1 de T125 A2.

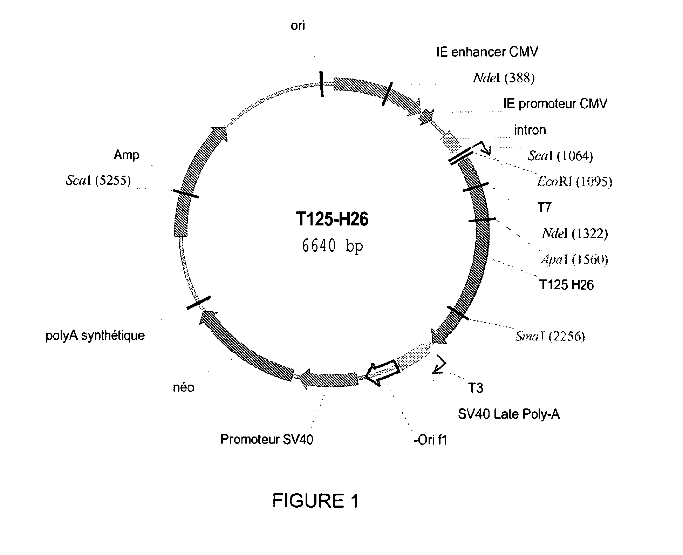

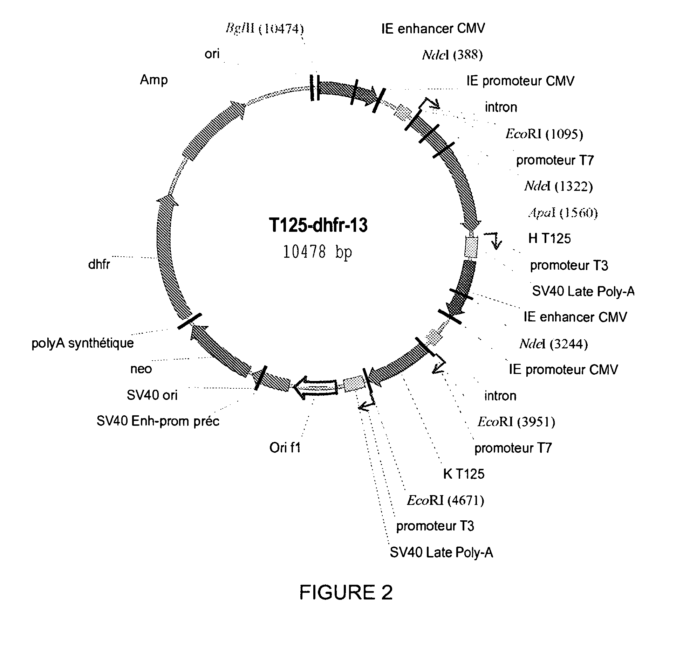

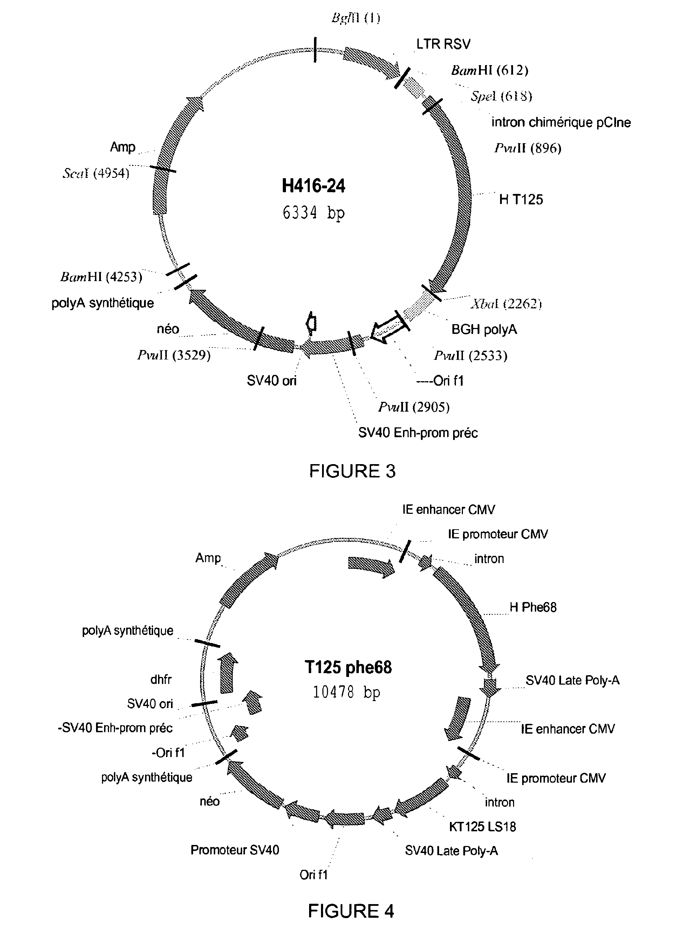

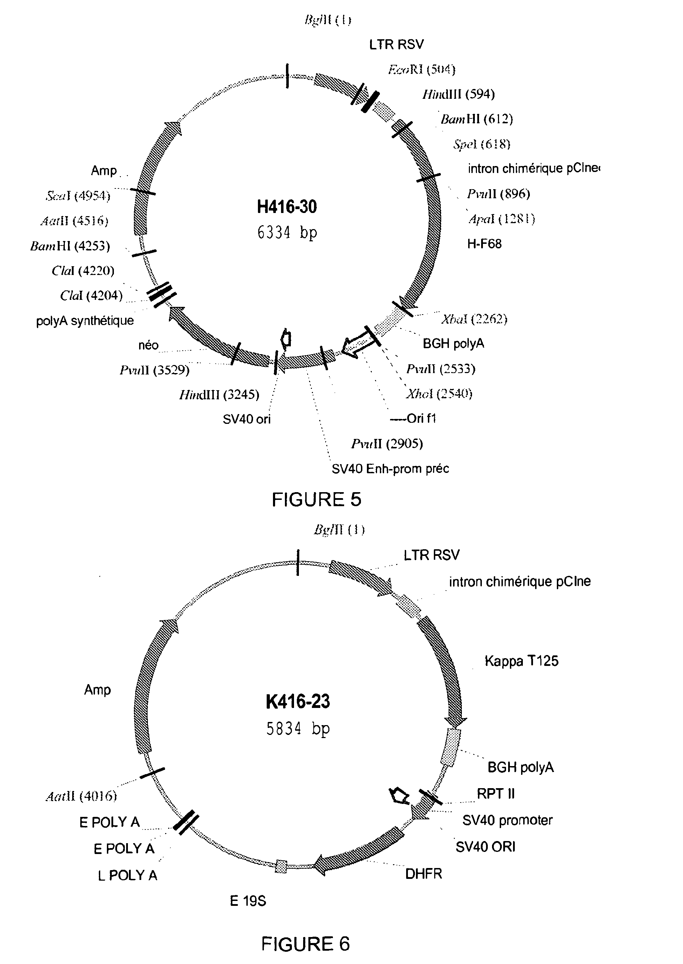

Les vecteurs utilisés étaient les suivants :
- Vecteur T125-H26
   Ce vecteur contient l'UT H du clone T125 A2 (cf. carte du vecteur en figure 1).
- Vecteur T125-DHFR
   Ce vecteur contient les UT H et Kappa du clone T125 A2 ainsi que DHFR (cf. carte du vecteur en figure 2).
- vecteur H416-24
   Ce vecteur intermédiaire chaîne lourde contient l'UT H du clone T125 A2 (cf. carte du vecteur en figure 3).
- H416-30
   Ce vecteur d'expression chaîne lourde présente la mutation C68F dans la région variable VH de l'anticorps. Il a été construit à partir du plasmide T125-Phe68 (carte en figure 4) porteur de la mutation C68F et d'un vecteur intermédiaire dépourvu d'intron (cf. carte en figure 5).
- vecteur K416-23
   Ce vecteur d'expression chaîne légère contient les UT Kappa du clone T125 A2 et DHFR (cf. carte en figure 6).

### Résultats :

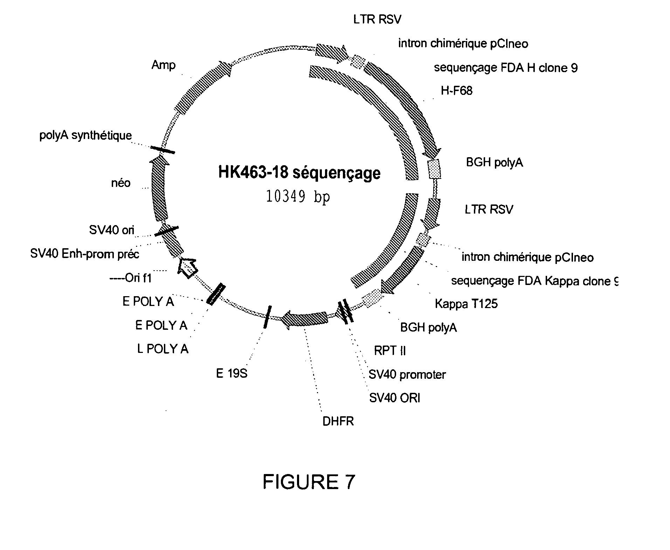

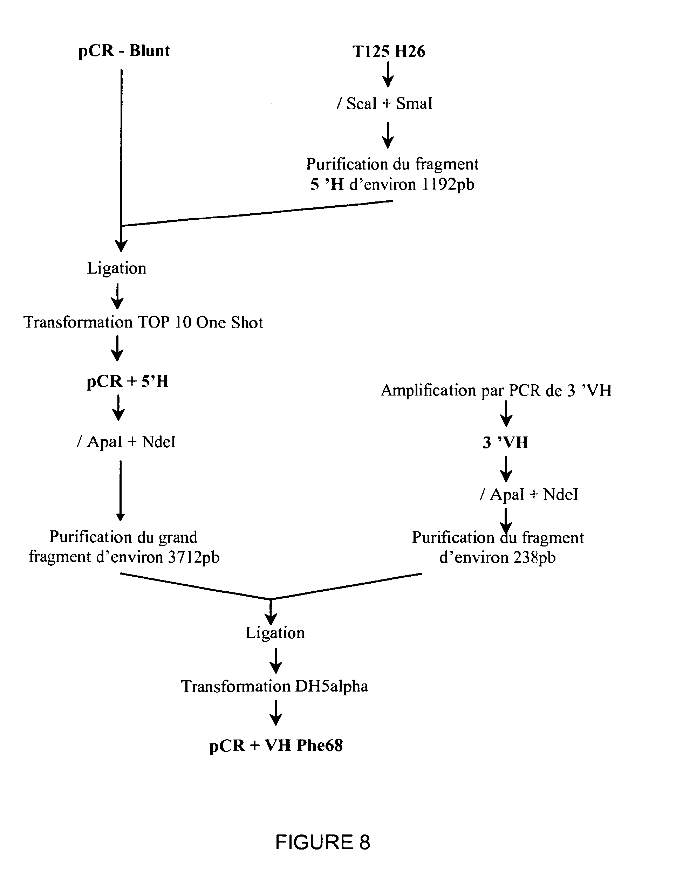

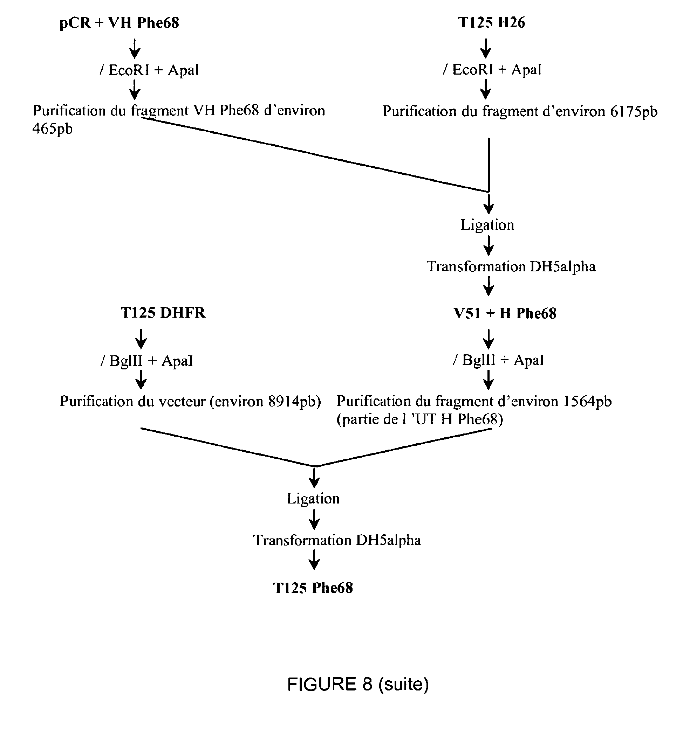

Le séquençage de l'anticorps anti-D d'origine R297, issu du clone T125 A2 (lymphocyte B issu d'un donneur immunisé transformé par l'EBV), a permis de montrer qu'il possède dans la région variable de sa chaîne lourde (VH) une cystéine en position 68 (position 67 selon la nomenclature de Kabat (Kabat et al., "Sequences of Proteins of Immunological Interest", NIH Publication, 91-3242 (1991) localisée dans la région charpente 3 (« framework » FWR3, selon Kabat). Cet anticorps présente donc un résidu cystéine additionnel en plus des deux cystéines en position 22 et 96 (position 22 et 92 selon la nomenclature de Kabat) engagées dans un pont disulfure.
La mutagenèse a été réalisée par la technique d'amplification par PCR d'un fragment au niveau de la région 3' de la séquence VH sur le vecteur chaîne lourde T125-H26 à l'aide des amorces décrites ci-dessus. Le codon TGC codant pour l'acide aminé cystéine a été substitué par le codon TTC codant pour l'acide aminé phénylalanine.
Ce fragment 3' de VH obtenu a été ligué avec le fragment 5' de VH dans un vecteur commercial intermédiaire (comportant le fragment 5' de VH issu de T125-H26). Le fragment VH Phe68 obtenu, correspondant au fragment VH muté, a ensuite été introduit dans le vecteur T125 DHFR pour former le vecteur d'expression final T125-Phe68 (cf schéma de la figure 8).
Le vecteur T125-Phe68 contient donc l'unité de transcription (UT) kappa de T125 A2 et l'UT H mutée (F68). Le contrôle de la présence de la mutation a été réalisé par séquençage sur quatre clones.
Le vecteur d'expression final HK463-18 (cf carte de la figure 7), contenant les deux unités de transcription (UT) H mutée (F68) et Kappa de l'anticorps anti-D T125 A2, a été construit par clonage du vecteur chaîne légère K416-23 (contenant les UT Kappa et DHFR) dans le vecteur chaîne lourde optimisé H416-30 (codant la chaîne lourde porteuse de la mutation C68F au niveau de sa région VH). Ces deux vecteurs d'expression sont déjà utilisés pour les co-transfections dans la lignée YB2/0.

### Exemple 3 : Caractérisation fonctionnelle de l'anticorps muté

Dans cette étude, l'impact de la mutation C68F a été évalué sur l'activité fonctionnelle de l'anticorps anti-D T125.

Le sang est prélevé sur des donneurs volontaires en tubes citrate de 7ml fournis par l'Etablissement Français du Sang (EFS) de Rungis
- Hématies Rhésus D négatif (groupes ABO indifférents)
- Hématies Rhésus D positif groupe O R1 R1 (densité antigénique optimale)

### 3. 1 Etude des fonctions liées spécifiquement à la partie Fab

### Etude de la spécificité de reconnaissance de l'épitope D

Aucune fixation des anticorps anti-D non muté (C68) et muté (F68) n'a été observée sur les hématies de donneurs Rhésus négatif en comparaison à un témoin correspondant à l'autofluorescence non spécifique dans les conditions de réalisation du dosage. Ceci confirme la spécificité de reconnaissance de l'épitope D de ces deux anticorps.

### Mesure de l'activité spécifique de l'anti-D par cytométrie

Les activités spécifiques des deux anticorps non muté (C68) et muté (F68) sont identiques (avec un intervalle de confiance de 15%). La fonctionnalité du domaine Fab est comparable pour les deux anticorps testés.

Etude de compétition in vitro par cytométrie entre des hématies 0+ R1R1 saturées et non saturées par les anticorps anti-D
Dans les conditions expérimentales de compétition, on peut conclure que les constantes de dissociation obtenues pour les trois anticorps testés sont équivalentes. Il n'y a donc pas d'impact de la mutation C68F sur la constante de dissociation.

### Conclusion

D'après ces résultats, la spécificité de reconnaissance antigénique et l'activité spécifique anti-D sont identiques pour les deux anticorps anti-D non muté (C68) et muté (F68). La fonctionnalité du domaine Fab (site de reconnaissance de l'antigène à l'anticorps) n'apparaît donc pas modifiée par la mutation C68F.

### 3. 2 Tests fonctionnels in vitro des parties Fc et Fab de l'anti-D

Les anticorps anti-D non muté (C68) et muté (F68) ont été évalués dans deux tests mesurant la fixation des anticorps anti-D à l'antigène et l'engagement de leur partie Fc avec le CD16 (récepteur FcgRIII).

### Activité ADCC (antibody-dependent cellular cytotoxicity)

Il n'y a pas de différences majeures entre l'activité ADCC des anticorps non mutés (C68) par rapport à celle des anticorps mutés (F68), le pourcentage de lyse obtenu de tous ces anticorps se situant entre l'activité de l'anticorps R297 et celle de l'anticorps polyclonal WinRho. Une modélisation des courbes avec le logiciel PRISM a été requise pour comparer plus précisément l'activité des anticorps.
Les valeurs obtenues de Emax (concentration d'anticorps correspondant à l'activité maximale) sont respectivement de 46 +/- 7 ng/ml et de 48 +/- 2 ng/ml pour les anticorps non mutés (C68) et mutés (F68). Les EC50 (concentration d'anticorps requise pour obtenir 50% de l'activité maximale) sont respectivement de 20 +/- 3 ng/ml et de 21 +/- 2 ng/ml pour les anticorps non mutés (C68) et mutés (F68).
La modélisation des courbes et l'expression des valeurs de Emax et d'EC50 indiquent que la mutation C68F n'a pas d'effet sur l'activité ADCC des anticorps.

### Activation du CD16

Les pourcentages d'activation du CD16 obtenus sont respectivement de 104 +/- 7% et 100 +/- 16% pour les anticorps non mutés (C68) et les anticorps mutés (F68).

### Conclusion

D'après ces résultats, l'activité ADCC et l'activation du CD16 ne sont pas modifiées par la mutation C68F.

Les résultats des différents essais réalisés montrent que les fonctions de l'anticorps anti-D T125 A2 supportées par les parties Fab (spécificité, activité spécifique, dissociation) et Fc (ADCC, activation CD16) ne sont pas modifiées par la mutation C68F.

### Exemple 4 : Etude structurale comparative des clones non mutés et mutés

### Les glycannes des anticorps anti-D non mutés (C68) et mutés (F68) ont été analysés par HPCE-LIF.

Les résultats des différentes cartes glycanniques des anticorps anti-D non mutés (C68) et mutés (F68) montrent des profils comparables avec des formes biantennées non sialylées agalactosylées fucosylées ou non (G0F, G0) et monogalactosylées fucosylées ou non (G1F, G1). Les formes majoritaires sont toujours de type agalactosylées (G0). Des différences sont observées dans le taux des formes fucosylées qui apparaît plus faible pour les anticorps anti-D mutés (F68). Ces derniers présentent également des structures possédant une N-acetylglucosamine (GlcNAc) en position bissectrice, c'est-à-dire entre les deux antennes. Ces structures GlcNAc en position bissectrice sont absentes sur les anticorps anti-D non mutés (C68).
Ces différences de structure au niveau des taux de fucose et de GlcNAc en position bissectrice n'affectent pas l'activité ADCC et l'activation CD16. La différence de profil de glycosylation n'est probablement pas liée à la mutation. En effet, les études réalisées sur différents anticorps monoclonaux produits dans la lignée YB2/0 ont montré des profils de glycosylation très variable en fonction des clones étudiés mais aussi en fonction du temps de culture sur un même clone.

La mutation de la Cystéine par une Phénylalanine en position 68 (C68F) de la région variable de la chaîne lourde du clone T125 A2 a été réalisée par PCR. La séquence de la région variable de la chaine lourde, porteuse de la mutation ponctuelle G à T, a été amplifiée à partir du plasmide T125-Phe68 puis clonée dans un vecteur chaîne lourde optimisé H416-30. Au final, un vecteur d'expression unique HK463-18 a été construit à partir du vecteur chaine lourde H416-30 et du vecteur chaîne légère K416-23. La présence de la mutation a été vérifiée par séquençage en qualité FDA. L'anticorps muté F68 issu de ce vecteur unique peut donc être produit dans la lignée YB2/0.

Les résultats d'analyse fonctionnelle ont permis de montrer que la spécificité de reconnaissance de l'antigène, l'activité spécifique anti-D et la constante de dissociation ne sont pas modifiées par la mutation C68F. De plus, l'activité ADCC et la capacité d'activation du CD16 ne sont pas affectées.

Des différences structurales entre les clones non mutés (C68) et mutés (F68) ont été observées. Le taux de fucosylation est plus faible et une N-acetylglucosamine (GlcNAc) est présente en position bissectrice pour les clones mutés (F68). Cette différence de profil de glycosylation ne modifie pas les fonctionnalités de l'anticorps et n'est donc probablement pas liée à la mutation C68F.

En conclusion, l'anticorps anti-D de l'invention possédant la mutation C68F présente une fonctionnalité comparable à l'anticorps R297 du clone T125 A2.

### Exemple 5 : Formulations de l'anticorps

Les formulations suivantes ont été préparées.

**Tableau 1. Formulation de l'anticorps avec Tween® 80 :**

| **Ingrédient** | **Concentration** |
|---|---|
| Anticorps de l'invention | 0.3g/L |
| Tampon citrate | 30mM |
| Mannitol | 17g/L |
| NaCl | 3,25g/L |
| Polysorbate 80 (Tween® 80) | 400ppm |

| | |
|---|---|
| pH=6,5 | |

**Tableau 2. Formulation de l'anticorps avec Lutrol ® F 68 :**

| **Ingrédient** | **Concentration** |
|---|---|
| Anticorps de l'invention | 0.3g/L |
| Tampon citrate | 30mM |
| Mannitol | 17g/L |
| NaCl | 3,25g/L |
| Poloxamer 188 (Lutrol ® F 68) | 301 ppm |

| | |
|---|---|
| pH=6,5 | |

Une étude de la stabilité des formulations a été menée sur plusieurs mois. L'étude a inclus l'inspection visuelle régulière des flacons contenant la formulation (pour vérifier la couleur, l'opalescence et la formation éventuelle de particules), la vérification du pH, de l'osmolalité, la vérification d'une dégradation de l'anticorps (par SDS PAGE en conditions réduites ou non réduites), la vérification de l'agrégation éventuelle des anticorps. Des tests de pureté ont été aussi réalisés par IEF (isoélectrofocus) et HPSEC (chromatographie par exclusion de taille à haute performance). L'état oxydatif est recherché par RP-HPLC.

Les formulations sont stables après 12 mois à 5°C, et jusqu'à au moins 4 mois à 25°C.
## Revendications

1. Anticorps monoclonal anti-RhD, qui est une immunoglobuline IgG1 tétramérique composée de deux chaînes lourdes et deux chaînes légères, la chaîne lourde comprenant la séquence d'acides aminés SEQ ID N°2, et la chaîne légère comprenant la séquence d'acides aminés SEQ ID N°4.

2. Procédé de production d'un anticorps de la revendication 1, ledit procédé comprenant les étapes suivantes : a) la culture dans un milieu et conditions de culture appropriés d'une cellule hôte exprimant une chaîne lourde d'anticorps comprenant la séquence SEQ ID N°2, et une chaîne légère d'anticorps comprenant la séquence SEQ ID N°4 ; et b) la récupération desdits anticorps ainsi produits à partir du milieu de culture ou desdites cellules cultivées.

3. Anticorps de la revendication 1, à titre de médicament.

4. Composition pharmaceutique comprenant l'anticorps de la revendication 1, en association avec des excipients pharmaceutiquement acceptables.

5. Composition selon la revendication 4, comprenant un tampon citrate.

6. Composition selon la revendication 4 ou 5, comprenant un tensioactif non ionique.

7. Composition selon l'une des revendications 4 à 6, comprenant un tampon citrate 30mM, pH 6,5, du polysorbate 80 ou un poloxamer, du mannitol et du NaCl.

8. Composition selon la revendication 7, comprenant un tampon citrate 30mM, pH6,5, 400ppm de polysorbate 80, 17g/L de mannitol et 3,25g/L de NaCl.

9. Composition selon la revendication 7, comprenant un tampon citrate 30mM, pH6,5, 301 ppm de poloxamer 188, 17g/L de mannitol et 3,25g/L de NaCl.

10. Composition selon l'une des revendications 7 à 9, dans laquelle la concentration en anticorps est de 0,3g/L.

11. Anticorps de la revendication 1, pour une utilisation pour la prévention d'une allo-immunisation Rhésus d'individus Rh négatifs.

12. Anticorps pour une utilisation selon la revendication 11, pour la prévention d'une maladie hémolytique chez le nouveau-né, par administration à une femme RhD négatif.

13. Anticorps selon la revendication 1, pour une utilisation dans la prévention ou le traitement d'un Purpura Thrombocytopénique Idiopathique (PTI).

## Patentansprüche

1. Monoklonaler Anti-Rhesus-D-Antikörper, der ein aus zwei schweren Ketten und zwei leichten Ketten zusammengesetztes tetrameres IgG1-Immunglobulin ist, wobei die schwere Kette die Aminosäuresequenz SEQ ID NR: 2 umfasst und die leichte Kette die Aminosäuresequenz SEQ ID NR: 4 umfasst.

2. Verfahren zur Herstellung eines Antikörpers nach Anspruch 1, wobei besagtes Verfahren folgende Schritte umfasst: a) die Kultivierung in einem Medium und unter geeigneten Bedingungen einer Wirtszelle, die eine schwere Antikörper-Kette, umfassend die Sequenz SEQ ID NR: 2, und eine leichte Antikörper-Kette, umfassend die Sequenz SEQ ID NR: 4, exprimiert, und b) die Wiedergewinnung besagter derartig produzierter Antikörper aus dem Kulturmedium oder aus besagten kultivierten Zellen.

3. Antikörper nach Anspruch 1 als Medikament.

4. Pharmazeutische Zusammensetzung, umfassend den Antikörper nach Anspruch 1 in Kombination mit pharmazeutisch verträglichen Arzneimittelträgern.

5. Zusammensetzung gemäß Anspruch 4, umfassend einen Citrat-Puffer.

6. Zusammensetzung gemäß Anspruch 4 oder 5, umfassend ein nichtionisches Tensid.

7. Zusammensetzung gemäß einem der Ansprüche 4 bis 6, umfassend 30 mM Citrat-Puffer, pH 6,5, Polysorbat 80 oder ein Poloxamer, Mannit und NaCl.

8. Zusammensetzung gemäß Anspruch 7, umfassend 30 mM Citrat-Puffer, pH 6,5, 400 ppm Polysorbat 80, 17 g/l Mannit und 3,25 g/l NaCl.

9. Zusammensetzung gemäß Anspruch 7, umfassend 30 mM Citrat-Puffer, pH 6,5, 301 ppm Poloxamer 188, 17 g/l Mannit und 3,25 g/l NaCl.

10. Zusammensetzung gemäß einem der Ansprüche 7 bis 9, in der die Antikörper-Konzentration 0,3 g/l beträgt.

11. Antikörper nach Anspruch 1 zur Verwendung für die Prävention einer Rhesus-Alloimmunisierung von Rh-negativen Individuen.

12. Antikörper zur Verwendung gemäß Anspruch 11 für die Prävention einer hämolytischen Krankheit beim Neugeborenen mittels Verabreichung an eine RhD-negative Frau.

13. Antikörper gemäß Anspruch 1 zur Verwendung bei der Prävention oder Behandlung einer idiopathischen thrombozytopenischen Purpura (ITP).

## Claims

1. An anti-RhD monoclonal antibody, which is a tetrameric IgG1 immunoglobulin composed of two heavy chains and two light chains, wherein the heavy chain comprises amino acid sequence SEQ ID NO : 2, and the light chain comprises amino acid sequence SEQ ID NO:4.

2. A method for producing an antibody of claim 1, wherein said method comprises the following steps : a) culturing a host cell expressing an antibody heavy chain comprising sequence SEQ ID NO : 2 and an antibody light chain comprising sequence SEQ ID NO:4, in suitable medium and culture conditions; and b) collecting said antibodies so produced from the culture medium or from said cultured cells.

3. The antibody of claim 1, as a medicament.

4. A pharmaceutical composition comprising the antibody of claim 1, in association with pharmaceutically acceptable excipients.

5. The composition according to claim 4, comprising a citrate buffer.

6. The composition according to claim 4 or 5, comprising a nonionic surfactant.

7. The composition according to any of claims 4 to 6, comprising a 30mM citrate buffer, pH 6.5, polysorbate 80 or poloxamer, mannitol and NaCl.

8. The composition according to claim 7, comprising a 30mM citrate buffer, pH 6.5, 400ppm polysorbate 80, 17g/L mannitol and 3.25g/L NaCl.

9. The composition according to claim 7, comprising a 30mM citrate buffer, pH 6.5, 301 ppm polysorbate 188, 17g/L mannitol and 3.25g/L NaCl

10. The composition according to any of claims 7 to 9, wherein the antibody concentration is 0.3g/L.

11. The antibody of claim 1, for use in preventing Rhesus allo-immunisation of Rh negative individuals.

12. The antibody for use according to claim 11, for preventing a hemolytic disease in new-born, by administration to a RhD negative woman.

13. The antibody of claim 1, for use in preventing or treating Idiopathic Thrombocytopenic Purpura (ITP).
